# EUROPEAN PATENT APPLICATION

(11) **EP 1 911 745 A1**
(43) Date of publication of application: **16.04.2008**
(21) Application number: 06768369.8
(22) Date of filing: 24.07.2006
(51) Int. Cl.: C07D 211/32

(54) **PROCESS FOR PRODUCING 1-BENZYL-4-[(5,6-DIMETHOXY-1-INDANONE)-2-YLIDENE]METHYLPIPERIDINE**

(30) Priority: 25.07.2005 JP 2005213820
(71) Applicant: Eisai R&D Management Co., Ltd., Tokyo 112-8088 (JP)
(72) Inventor: IMAI, Akio Eisai Co., Ltd., Kashima-gun, Ibaraki 314-0255 (JP); SHIMOTANI, Akihiko Eisai Co., Ltd., Kashima-gun, Ibaraki 314-0255 (JP); TSURUGI, Tomio Eisai Co., Ltd., Kashima-gun, Ibaraki 314-0255 (JP); NARABE, Yukio Eisai Co., Ltd., Kashima-gun, Ibaraki 314-0255 (JP)
(74) Representative: HOFFMANN EITLE
(86) International application number: PCT/JP2006/314568
(87) International publication number: WO 2007/013395

(57) **Abstract**

It is intended to provide a process for producing 1-benzyl-4-[(5,6-dimethoxy-1-indanon)-2-ylidene]methylpiperidine represented by the structural formula (I): (I) which is useful as an intermediate material for a pharmaceutical or a solvate thereof in which impurity content is further reduced and operation is simple and suitable for industrial production by reacting 5,6-dimethoxy-1-indanone with 1-benzyl-4-formylpiperidine in a reaction solvent in the presence of a base and then gradually crystallizing a target substance in a reaction mixture in a high temperature region.

## Description

### TECHNICAL FIELD

The present invention relates to a process for producing 1-benzyl-4-[(5,6-dimethoxy-1-indanon)-2-ylidene]methylpiperidine useful as an intermediate material for a pharmaceutical, or a solvate thereof. More specifically, the present invention relates to a process for producing 1-benzyl-4-[(5,6-dimethoxy-1-indanon)-2-ylidene]methyl-piperidine or a solvate thereof in high purity by reacting 5,6-dimethoxy-1-indanone with 1-benzyl-4-formylpiperidine in a solvent in the presence of a base, followed by gradual crystallization in the reaction mixture in a high temperature region. [1-Benzyl-4-[(5,6-dimethoxy-1-indanon)-2-yl]methylpiperidine hydrochloride (common name: donepezil hydrochloride)] produced by subjecting 1-benzyl-4-[(5,6-dimethoxy-1-indanon)-2-ylidene]methylpiperidine or a solvate thereof to catalytic hydrogenation by the method described in JP-A-1-79151, J. Med. Chem, 38, 4821 (1995) or International Patent Laid-Open No. WO2005/105742 pamphlet to obtain 1-benzyl-4-[(5,6-dimethoxy-1-indanon)-2-yl]methylpiperidine and reacting this compound with, for example, hydrochloric acid is effective for the treatment, prevention, remission, amelioration and the like of, for example, various senile dementias such as Alzheimer type senile dementia, etc.; cerebrovascular accidents associated with, for example, a cerebral accident (e.g. cerebral hemorrhage or cerebral infarction), cerebral arteriosclerosis, or an external wound in head; and hypoprosexia, lalopathy, hypobulia, emotional disturbance, retention defect, hallucinosis-paranoia and behavior abnormality which are associated with, for example, encephalitis or cerebral palsy.

### BACKGROUND ART

1-Benzyl-4-[(5,6-dimethoxy-1-indanon)-2-ylidene]methylpiperidine represented by the formula (I) : 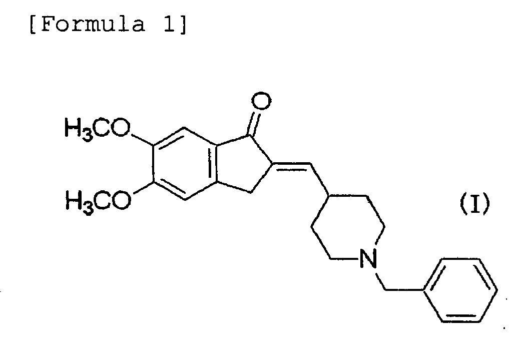 which is the desired compound in the production process of the present invention is a well-known compound and is known to be producible by reacting 1-benzyl-4-formylpiperidine represented by the formula (II): 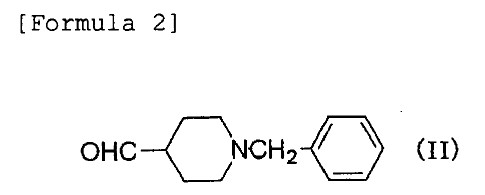 with 5,6-dimethoxy-l-indanone represented by the formula (III): 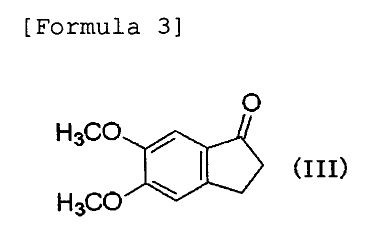 in the presence of a base (see, for example, patent document 1, patent document 2 and patent document 3).

Patent document 1 discloses a reaction scheme represented by the following general formulas in regard to a process for producing the compound of the formula (I) (the left-hand top column on page 17 of patent document 1): 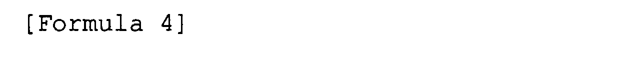 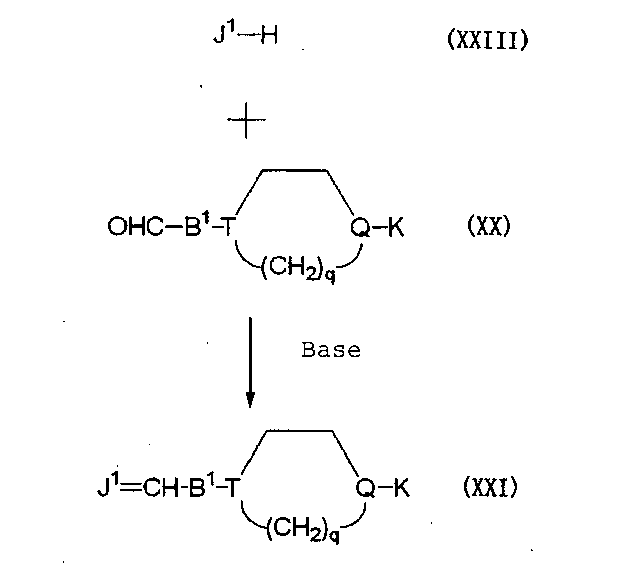 In the above reaction scheme, a compound such as a substituted or unsubstituted indanone represented by the general formula (XXIII) and an aldehyde represented by the general formula (XX) are subjected to aldol condensation by a conventional method to produce a compound of the general formula (XXI), one of desired substances. Specifically, patent document 1 describes the following: the compound of the general formula (XXI) may be produced by adding the compound of the general formula (XXIII) to lithium diisopropylamide at about -80°C in a solvent such as tetrahydrofuran, adding thereto the aldehyde represented by the general formula (XX) at the same temperature to carry out the reaction, and then warming the reaction solution to room temperature to carry out dehydration, and the compound of the general formula (XXI) may be produced also by a process of dissolving the compound of the general formula (XX) and the compound of the general formula (XXIII) in a solvent such as tetrahydrofuran, adding thereto a base such as sodium methylate at about 0°C, and then carrying out the reaction at room temperature (from the second line from the bottom in the left-hand top column on page 17 of patent document 1 to the fourth line from the bottom in the right-hand top column on page 17).

In addition, condensation reactions corresponding to the above-mentioned reactions are described in working examples in patent document 1 as follows: in Example 3(b), Example 22, Example 34 and Example 36, there is described a case of using anhydrous tetrahydrofuran as a solvent for reaction, adding indanone to lithium diisopropylamide at -78°C, adding an aldehyde thereto at the same temperature, slowly raising the temperature, and then carrying out the reaction with stirring at room temperature, and in Example 28, there is described a case of adding a 28% sodium methylate/methanol solution to an aldehyde and indanone in anhydrous tetrahydrofuran at 0°C and then carrying out the reaction with stirring at room temperature.

Patent document 2 discloses a reaction scheme represented by the following formulas in regard to a process for producing the compound of the formula (I) (page 5 of patent document 2): 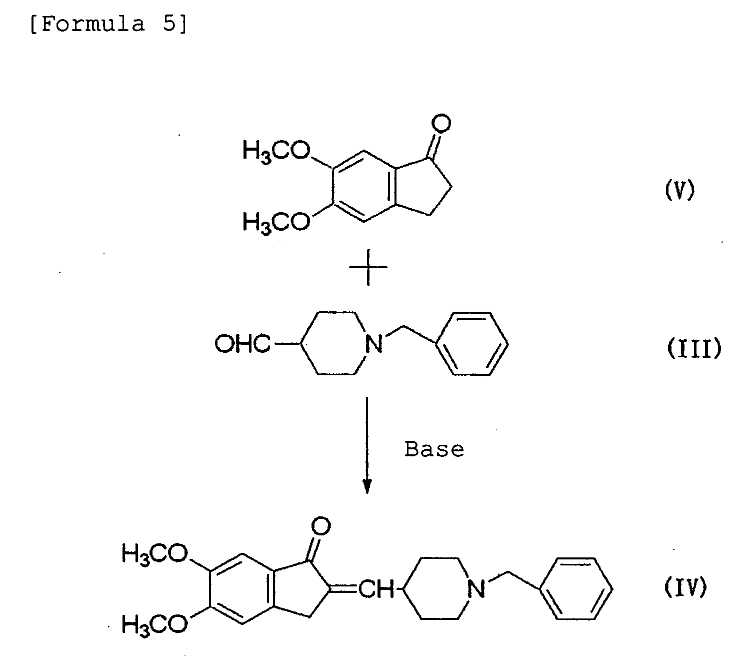 In the above reaction scheme, 5,6-dimethoxyindanone represented by the formula (V) and 1-benzyl-4-formylpiperidine represented by the formula (III) are subjected to aldol condensation by a ' conventional method to produce a compound of the formula (IV). Specifically, patent document 2 describes the following: the compound of the formula (IV) may be produced by producing lithium diisopropylamide in a solvent such as tetrahydrofuran, adding thereto 5,6-dimethoxyindanone represented by the formula (V) at about -80°C, adding thereto 1-benzyl-4-formylpiperidine represented by the formula (III) at the same temperature to carry out the reaction, and then warming the reaction solution to room temperature to carry out dehydration, and the compound of the formula (IV) may be produced also by a process of dissolving the compound of the formula (V) and the compound of the formula (III) in a solvent such as tetrahydrofuran, adding thereto a base such as sodium methylate at about 0°C, and carrying out the reaction at room temperature (from the first line in the left-hand column on page 5 of patent document 2 to the sixth line in the right-hand column on page 5).
In Production Example 1 (b) in patent document 2, there is described a case of using anhydrous tetrahydrofuran as a solvent for reaction, adding indanone to lithium diisopropylamide at -78°C, adding an aldehyde thereto at the same temperature, slowly raising the temperature, and then carrying out the reaction with stirring at room temperature (from the eighth line to the fifteenth line in the left-hand column on page 8 of patent document 2).

Patent document 3 discloses a reaction scheme represented by the following formulas in regard to a process for producing the compound of the formula (I) (the thirteenth paragraph on page 3 of patent document 3) : 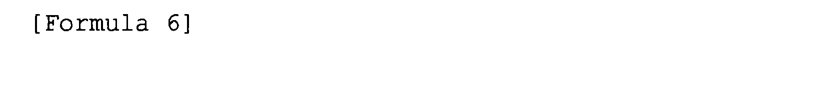 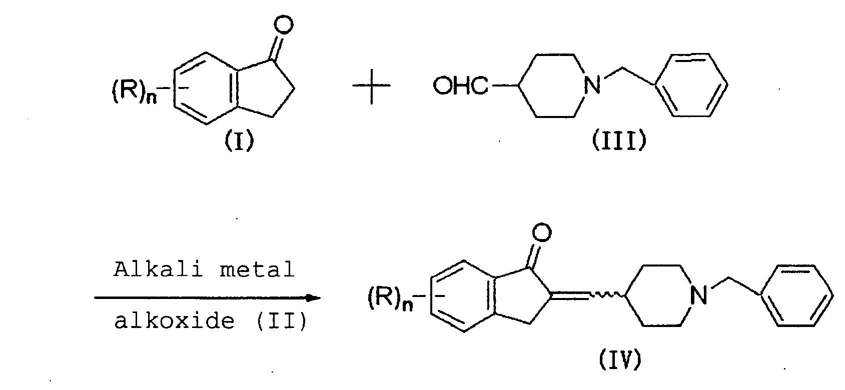 In the above reaction scheme, a 1-benzyl-4-[(1-indanon)-2-ylidene]methylpiperidine derivative (IV) is produced by reacting a 1-indanone derivative (I) with 1-benzyl-4-formylpiperidine (III) in the presence of an alkali metal alkoxide (II). Specifically, patent document 3 describes the following: after addition or dropwise addition of the alkali metal alkoxide to any of various inert organic solvents containing the 1-indanone derivative (I) and 1-benzyl-4-formylpiperidine (III) at room temperature or higher, the reaction is carried out at room temperature to 70°C (the twenty-first paragraph on page 4 of patent document 3).
Patent document 1: JP-A-1-79151
Patent document 2: Japanese Patent No. 2578475
Patent document 3: JP-A-11-171861

### DISCLOSURE OF THE INVENTION

### Problem to be Solved by the Invention

In the above-mentioned process of producing the compound of the formula (I) by the aldol condensation reaction in any of patent documents 1 to 3, by-products including compounds represented by the formula (a) and the formula (b): 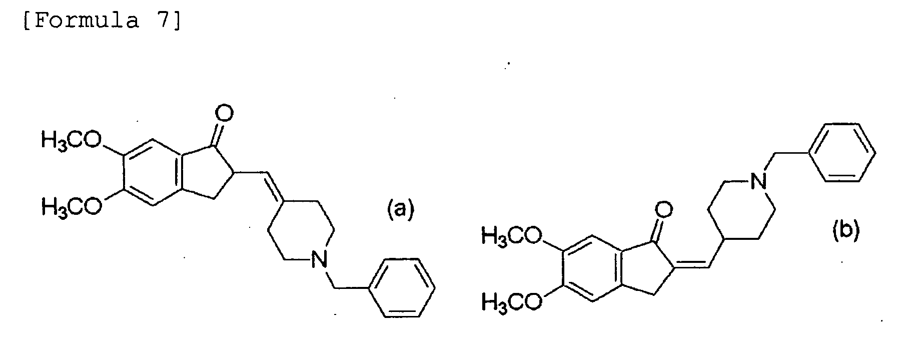 are formed, so that a well-known purifying means such as column chromatography or recrystallization is considered necessary in order to subject the reaction product to a subsequent reaction.
Of these by-products, the compound of the formula (a), in particular, is very difficult to remove even by subjecting the crude compound of the formula (I) to the purifying means such as column chromatography or recrystallization. Moreover, the reaction of the compound of the formula (a) does not proceed easily in catalytic hydrogenation, a subsequent step, so that this compound remains as it is as an impurity.
In patent document 3, since the desired compound is isolated after adding water to the reaction solution or cooling the reaction solution, the purity of the compound of the formula (I) obtained is not always sufficient, and further purification results in a decreased yield and troublesome operations.

In patent documents 1 and 2, since lithium diisopropylamide having a very high reactivity is used, it is necessary to maintain the reaction system at an extremely low temperature and assure nonaqueous conditions. Thus, such a production process is unsuitable for industrial production.
Therefore, a process for producing the compound of the formula (I) is desired which reduces the production of the by-products, comprises easy operations and is suitable for industrial production.

### Means for Solving the Problem

The present inventors earnestly investigated in order to solve the above problem, and consequently found that by reacting the compound of the formula (III) with the compound of the formula (II) in a solvent in the presence of a base and then gradually crystallizing the desired compound in the reaction mixture in a high temperature region, a process for producing the compound of the formula (I) or a solvate thereof is attained which reduces the production of the by-products, comprises easy operations and is suitable for industrial production. Thus, the present invention has been accomplished.

That is, the present invention relates to the following items 1) to 18).
1) A process for producing 1-benzyl-4-[(5,6-dimethoxy-1-indanon)-2-ylidene]methylpiperidine represented by the formula (I): 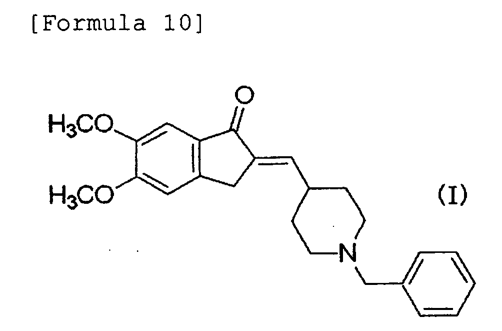 or a solvate thereof, characterized by reacting 5,6-dimethoxy-1-indanone represented by the formula (III): 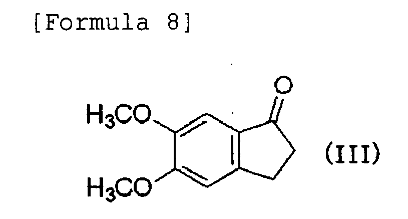 with 1-benzyl-4-formylpiperidine represented by the formula (II): 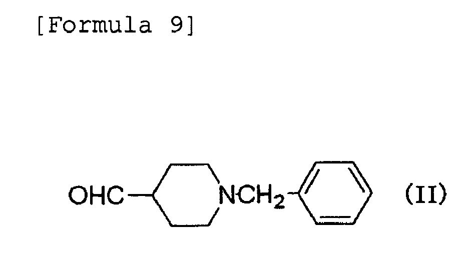 in a solvent in the presence of a base, and then gradually crystallizing the desired compound in the reaction mixture in a high temperature region.
2) A production process according to the above item 1), wherein the solvent is that selected from the group consisting of methanol, ethanol, 2-propanol, tetrahydrofuran, 1,2-dimethoxyethane, toluene, and mixed solvents thereof.
3) A production process according to the above item 1) or 2), wherein the solvent is methanol, ethanol, tetrahydrofuran, toluene, or a mixed solvent thereof.
4) A production process according to any one of the above items 1) to 3), wherein the solvent is methanol or ethanol.
5) A production process according to any one of the above items 1) to 3), wherein the solvent is tetrahydrofuran.
6) A production process according to any one of the above items 1) to 3), wherein the solvent is a mixed solvent of methanol and toluene and the mixing ratio of methanol to toluene is 8 : 2 to 2 : 8.
7) A production process according to any one of the above items 1) to 3), wherein the solvent is a mixed solvent of ethanol and toluene and the mixing ratio of ethanol to toluene is 8 : 2 to 2 : 8.
8) A production process according to any one of the above items 1) to 7), wherein the base is an alkali metal hydroxide or an alkali metal alkoxide.
9) A production process according to any one of the above items 1) to 8), wherein the base is sodium hydroxide or sodium methoxide.
10) A production process according to any one of the above items 1) to 9), wherein the base is sodium methoxide.
11) A production process according to any one of the above items 1) to 10), wherein the reaction temperature is 20°C to the reflux temperature of the solvent for reaction.
12) A production process according to any one of the above items 1) to 11), wherein the compound of the formula (I) or a solvate thereof is obtained while reducing the production of isomers of the desired compound by gradually crystallizing the desired compound in the reaction mixture in a high temperature region.
13) A production process according to the above item 12), wherein the isomer of the desired compound is a compound represented by the formula (a) or the formula (b): 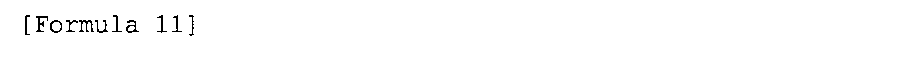 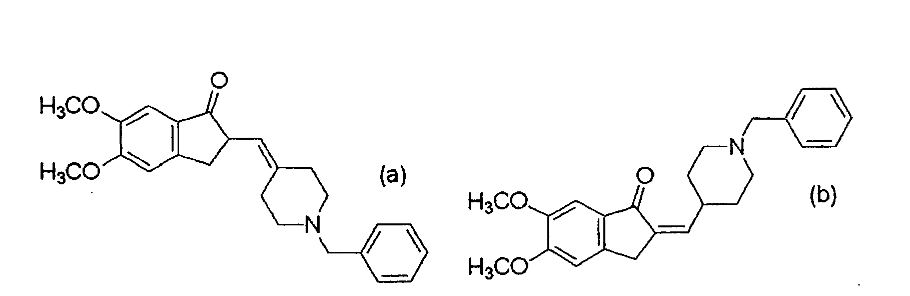
14) A production process according to any one of the above items 1) to 13), wherein the high temperature region is a region of 20°C to 80°C.
15) A production process according to any one of the above items 1) to 14), wherein the desired compound is crystallized by slowly cooling the reaction mixture from the reaction temperature in the high temperature region to a low temperature region.
16) A production process according to the above item 15), wherein the rate of cooling from the reaction temperature in the high temperature region to a low temperature region is 1°C/hour to 30°C/hour.
17) A production process according to any one of the above items 1) to 14), wherein the desired compound is crystallized while maintaining the reaction mixture at temperatures in the high temperature region, and then further crystallized by slowly cooling the reaction mixture to a low temperature region.
18) A production process according to the above item 17), wherein the rate of cooling from the high temperature region to a low temperature region is 1°C/hour to 60°C/hour.

The process for producing the compound of the formula (I) or a solvate thereof of the present invention is explained below in detail.

The compound of the formula (I) or a solvate thereof may be produced by reacting the compound of the formula (III) with the compound of the formula (II) in a solvent in the presence of a base and then gradually crystallizing the desired compound in the reaction mixture in a high temperature region.
All of the compound of the formula (I), the compound of the formula (II) and the compound of the formula (III) are well known and may be produced, for example, by the processes described in patent document 1, 2 or 3. As the compound of the formula (III), for example, a commercial product of Aldrich Chemical Co. (Aldrich:14, 782-6) may be used.

The solvent used in the reaction is not particularly limited as long as it is usable in the condensation reaction of the compound of the formula (II) with the compound of the formula (III). The solvent includes, for example, alcohol solvents, ester solvents, ether solvents, aliphatic hydrocarbon solvents, aromatic hydrocarbon solvents, and mixed solvents thereof. The alcohol solvents include, for example, methanol, ethanol, 2-propanol and t-butanol. The ester solvents include, for example, methyl acetate and ethyl acetate. The ether solvents include, for example, diethyl ether, diisopropyl ether, t-butyl methyl ether, tetrahydrofuran, 1,2-dimethoxyethane and dioxane. The aliphatic hydrocarbon solvents include, for example, pentane, hexane, heptane and cyclohexane. The aromatic hydrocarbon solvents include, for example, benzene, toluene and xylene. Of these, methanol, ethanol, 2-propanol, tetrahydrofuran, 1,2-dimethoxyethane, toluene, and mixed solvents thereof are preferable. Here, the term "mixed solvents thereof" means mixtures of two or more solvents (in any ratio) selected from the group consisting of the solvents exemplified above. As the solvent, methanol, ethanol, tetrahydrofuran, toluene, mixed solvents thereof and the like are the most suitable. When a mixed solvent of methanol and toluene or a mixed solvent of ethanol and toluene is used, the mixing ratio may be varied depending on the amounts of the starting materials used, the kind and amount of the base used, the reaction conditions and the like. Specifically, the mixing ratio of methanol or ethanol to toluene is preferably 8 : 2 to 2 : 8, in particularly, 6 : 4 to 4 : 6.
Although the amount of the solvent used may be properly varied, it is preferably, for example, 5 to 30 times, in particular, 8 to 20 times, most preferably 10 to 14 times, as large as the volume of the compound of the structural formula (III).

The base used is not particularly limited as long as it is usable in the condensation reaction. For example, organic amines and alkali metal compounds such as alkali metal hydroxides, alkali metal carbonates, alkali metal amides, alkali metal cyanides, alkali metal C₁₋₄ alkoxides, etc. are preferable. In particular, the alkali metal hydroxides or the alkali metal C₁₋₄ alkoxides are preferable and alkali metal methoxides are the most suitable. The alkali metal C₁-₄ alkoxides may be used in a solid state or in the form of a solution thereof in a corresponding C₁₋₄ alcohol. Here, the alkali metal includes, for example, lithium, sodium, potassium and cesium. A preferable example thereof is sodium. The C₁₋₄ alkoxides include, for example, methoxide, ethoxide, propoxide and butoxide.
The amount of the base used may be properly varied and is preferably, for example, 0.6 to 1.8 equivalents, in particular, 1.0 to 1.4 equivalents, most preferably 1.1 to 1.3 equivalents, per equivalent of the compound of the formula (III).

The reaction temperature is not particularly limited and the reaction may be carried out at 20°C to the reflux temperature of the solvent. The reaction temperature is particularly preferably 50°C to the reflux temperature of the solvent, most preferably the reflux temperature of the solvent. The reaction is completed in usually 15 minutes to 3 hours, preferably 30 minutes to 1 hour and 30 minutes.

An atmosphere for the reaction is not particularly limited. The reaction is preferably carried out substantially in the absence of oxygen and is preferably carried out under an atmosphere of an inert gas such as nitrogen or argon. In particular, the reaction is most preferably carried out substantially in the absence of oxygen from the start of the reaction to the completion of crystallization.

The production process of the present invention is characterized by the following: after the reaction of the compound of the formula (II) with the compound of the formula (III), when the resulting compound of the formula (I) or a solvate thereof is isolated, the compound of the formula (I) or the solvate thereof in the reaction mixture in a high temperature region is gradually crystallized. The term "high temperature region" means a high temperature region advantageous for crystallizing the compound of the formula (I) or the solvate thereof in high purity. The high temperature region varies depending on the solvent used. Specifically, it is, for example, 20 to 80°C, preferably 30 to 80°C, in particular, 30 to 70°C.

As a method for gradually crystallizing the compound of the formula (I) or the solvate thereof in the reaction mixture in the high temperature region, there are exemplified the following methods A and B. The compound of the formula (I) or the solvate thereof may be crystallized by a proper combination of these methods.

### (1) Method A

In this method, the reaction mixture is slowly cooled from the reaction temperature to a low temperature region to precipitate crystals of the compound of the formula (I) or the solvate thereof in the high temperature region, whereby a sufficient time to precipitate the crystals in the high temperature region is assured. The cooling rate of the reaction mixture may be properly varied. Specifically, it is, for example, 1°C/hour to 30°C/hour, preferably 3°C/hour to 20°C/hour, in particular, 5°C/hour to 10°C/hour.

### (2) Method B

In this method, crystals of the compound of the formula (I) or the solvate thereof in the reaction mixture were sufficiently precipitated in a definite high temperature region, and then the reaction mixture was slowly cooled to a low temperature region to carry out further crystallization. This high temperature region refers to the temperature region described above. The period of time for the sufficient precipitation may be properly varied. Specifically, it is, for example, 10 minutes to 4 hours, preferably 20 minutes to 2 hours, in particular, 30 minutes to 1 hour. After completion of the precipitation in the high temperature region, the reaction mixture is slowly cooled to a low temperature region. Specifically, the cooling rate of the reaction mixture is, for example, 1°C/hour to 60°C/hour, preferably 1°C/hour to 30°C/hour, in particular, 5°C/hour to 10°C/hour.

In the above methods A and B, the term "low temperature region" means a temperature region which permits assurance of a sufficient yield, such as -30 to 20°C, preferably 0 to 20°C, in particular, 4 to 10°C. For the progress of the crystallization, a method of accelerating the crystallization, such as a stirring operation or the addition of seed crystals may be properly adopted.

The production process of the present invention is characterized in that the compound of the formula (I) or a solvate thereof may be produced in high purity and high yield by gradual crystallization in the reaction mixture in the high temperature region.
At completion of the condensation reaction, the compound of the formula (I) and its isomers represented by the compound of the formula (a) are in equilibrium and they exist in a definite presence ratio though the ratio varies depending on the reaction conditions such as the solvent for reaction. By the gradual crystallization in the reaction mixture in the high temperature region, the compound of the formula (I), the main component of the reaction mixture is crystallized at first. Then, the above-mentioned equilibrium is shifted in the remaining solution phase, and the compound of the formula (a) and the compound of the formula (b) are isomerized to the compound of the formula (I) and moreover, the compound of the formula (I) or a solvate thereof is crystallized. By slowly cooling the reaction mixture while repeating the above crystallization-isomerization cycle, the total amount of the by-products in the reaction mixture may be reduced. By such gradual crystallization in the reaction mixture in the high temperature region in which the isomerization is possible, the purity and yield of the compound of the formula (I) or solvate thereof finally obtained may be improved.

The amount of a solvent used for the crystallization is, for example, 3 to 30 times, preferably 5 to 10 times, most preferably 5 to 8 times, as large as the volume of the compound of the formula (I) or a solvate thereof. It is also possible to charge a proper amount of the solvent previously at the time of the reaction, and it is also possible to increase or decrease the amount of the solvent properly after completion of the condensation reaction. The compound of the formula (I) of high purity may be obtained by filtration after completion of the crystallization.

A solvate of the compound of the formula (I) may be formed depending on the solvent for reaction. The compound of the formula (I) or the solvate thereof may be used as it is in a subsequent reaction after being collected from the reaction mixture by filtration. When the compound or solvate thereof collected is dried, the drying is conducted at a temperature of, for example, 20 to 110°C, preferably 30 to 60°C, for, for example, 1 to 48 hours, preferably 2 to 24 hours, though these conditions are varied depending on the solvent for reaction used.

### BEST MODE FOR CARRYING OUT THE INVENTION

The present invention is illustrated in detail with reference to the following examples, which should not be construed as limiting the scope of the invention.

### Example 1

### Production of 1-benzyl-4-[(5,6-dimethoxy-1-indanon)-2-ylidene]methylpiperidine using a mixed solvent of methanol and toluene and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 ml of a mixed solvent (methanol: toluene = 16: 14). After the air in the system was replaced with nitrogen, 2.4 g (12.4 mmol, 1.2 equivalents) of a 28%-sodium methoxide/methanol solution was poured into the reaction solution at 64°C. After completion of the pouring, stirring was continued with refluxing for 1 hour to complete the reaction. Then, the reaction solution was air-cooled until crystals were precipitated (precipitation temperature: 45°C). After the precipitation, the reaction solution was cooled (cooling rate: 18°C/hour) and the crystals precipitated at 5°C or higher were collected by filtration. The crystals collected by filtration were washed with water (30 ml) and 6 mL of methanol and dried at 50°C (1 hour and 20 minutes) and then at 110°C (1 hour and 10 minutes) to obtain 3.53 g of the crystals of the title compound (yield: 89.9%).
¹H-NMR (400MHz,CDCl₃) δ(ppm)=1.60-1.72(4H, m), 2.03-2.09(2H, m), 2.29-2.37(1H, m), 2.92-2.95(2H, m), 3.53 (2H, s), 3.60(2H, d, J=2Hz), 3.93 (3H, s), 3.98(3H, s), 6.67(1H, dt, J=10, 2Hz), 6.90(1H, s), 7.25-7.34(6H, m)

### Example 2

### Production of 1-benzyl-4-[(5,6-dimethoxy-1-indanon)-2-ylidene]methylpiperidine 1/2 toluene solvate using a mixed solvent of methanol and toluene and sodium methoxide and adopting method A

The process of Example 1 was repeated except for conducting the drying of crystals at 50°C (1 hour and 20 minutes) only.
1 H-NMR (400MHz, CDCl₃) 6 (ppm) =1 . 58-1.72 (4H, m), 2.03-2.09(2H, m), 2.28-2.38(2.5H, m), 2.91-2.94(2H, m), 3.53(2H, s), 3.59(2H, d, J=2Hz), 3.93(3H, s), 3.97(3H, s), 6.67(1H, dt, J=10, 2Hz), 6.90(1H, s), 7.13-7.33(8.5H, m)
In TG-DTA (Thermogravimetry Differential Thermal Analysis) of the dried crystals, a weight loss corresponding to 1/2 toluene was recognized together with a heat absorption peak at about 100°C. In addition, these crystals and crystals obtained by further drying them at 110°C (1 hour and 10 minutes) showed different patterns, respectively, in powder X-ray diffraction.

### Example 3

### Production of 1-benzyl-4-[(5,6-dimethoxy-1-indanon)-2-ylidene]methylpiperidine using methanol and sodium methoxide and adopting method B

Into a 100-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of methanol. After the air in the system was replaced with nitrogen, 2.4 g (12.4 mmol, 1.2 equivalents) of a 28%-sodium methoxide/methanol solution was poured into the reaction solution with refluxing (66°C). After completion of the pouring, stirring was continued with refluxing for 1 hour to complete the reaction, whereby crystals were precipitated. The crystals began to be precipitated (precipitation temperature: 66°C) 2 minutes after the pouring. The reaction solution was cooled to 5°C (cooling rate: 26°C/hour) with ice water and the crystals precipitated at 5°C or higher were collected by filtration. The crystals collected by filtration were washed with water (30 ml) and 6 mL of methanol and dried at 50°C (drying time: 1 hour and 30 minutes) to obtain 3.69 g of the crystals of the title compound (yield: 93.9%). ¹H-NMR data of these crystals agreed with those obtained in Example 1.

### Example 4

### Production of 1-benzyl-4-[(5,6-dimethoxy-1-indanon)-2-ylidene]methylpiperidine using a mixed solvent of methanol and toluene and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 ml of a mixed solvent (methanol: toluene = 16: 14). After the air in the system was replaced with nitrogen, 2.4 g (12.4 mmol, 1.2 equivalents) of a 28%-sodium methoxide/methanol solution was poured into the reaction solution at 61°C. After completion of the pouring, stirring was continued at 54-63°C for 1 hour to complete the reaction. Then, the reaction solution was air-cooled until crystals were precipitated (precipitation temperature: 50°C). After the precipitation of the crystals, the reaction solution was cooled (cooling rate: 25°C/hour) and the crystals precipitated at 6°C or higher were collected by filtration. The crystals collected by filtration were washed with water (30 ml) and 6 mL of methanol and dried at 50°C (drying time: 3 hours and 10 minutes) to obtain 3.24 g of the crystals of the title compound (yield: 82.5%). ¹H-NMR data of these crystals agreed with those obtained in Example 1.

### Example 5

### Production of 1-benzyl-4-[(5,6-dimethoxy-1-indanon)-2-ylidene]methylpiperidine using a mixed solvent of methanol and toluene and sodium methoxide and adopting method B

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 mL of a mixed solvent (methanol : toluene = 16 : 14). After the air in the system was replaced with nitrogen, 2.4 g (12.4 mmol, 1.2 equivalents) of a 28%-sodium methoxide/methanol solution was poured into the reaction solution at 50°C. After the pouring, stirring was continued at 45-53°C for 1 hour to complete the reaction, whereby crystals were precipitated. The crystals began to be precipitated (precipitation temperature: 48°C) 47 minutes after the pouring. After the continuous stirring at 45-53°C for 1 hour, the reaction solution was cooled (cooling rate: 22°C/hour) and the crystals precipitated at 3°C or higher were collected by filtration. The crystals collected by filtration were washed with water (30 mL) and 6 mL of methanol and dried at 50°C (drying time: 3 hours and 10 minutes) to obtain 3.17 g of the crystals of the title compound (yield: 80.70). ¹H-NMR data of these crystals agreed with those obtained in Example 1.

### Example 6

### Production of 1-benzyl-4-[(5,6-dimethoxy-1-indanon)-2-ylidene]methylpiperidine using a mixed solvent of methanol and toluene and sodium methoxide and adopting method B

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 ml of a mixed solvent (methanol : toluene = 16 : 14). After the air in the system was replaced with nitrogen, 2.4 g (12.4 mmol, 1.2 equivalents) of a 28%-sodium methoxide/methanol solution was poured into the reaction solution at 28°C. After completion of the pouring, stirring was continued at 23-30°C for 4 hours and 31 minutes to complete the reaction, whereby crystals were precipitated. The crystals began to be precipitated (precipitation temperature: 26°C) 1 hour after the pouring. After the continuous stirring at 23-30°C for 4 hours and 31 minutes, the reaction solution was ice-cooled (cooling rate: 18°C/hour) and the crystals precipitated at 7°C or higher were collected by filtration. The crystals collected by filtration were washed with water (30 ml) and 6 mL of methanol and dried at 50°C (drying time: 1 hour and 23 minutes) to obtain 2.96 g of the crystals of the title compound (yield: 75.4%). ¹H-NMR data of these crystals agreed with those obtained in Example 1.

### Example 7

### Production of 1-benzyl-4-[(5,6-dimethoxy-1-indanon)-2-ylidene]methylpiperidine using tetrahydrofuran and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of tetrahydrofuran. After the air in the system was replaced with nitrogen, 2.4 g (12.4 mmol, 1.2 equivalents) of a 28%-sodium methoxide/methanol solution was poured into the reaction solution with refluxing (63°C). After completion of the pouring, stirring was continued with refluxing for 1 hour to complete the reaction. Then, the reaction solution was cooled to 4°C (cooling rate: 30°C/hour). Crystals began to be precipitated at 28°C. The crystals precipitated at 4°C or higher were collected by filtration, washed with water (30 ml) and 6 mL of methanol and then dried at 50°C (drying time: 2 hours and 40 minutes) to obtain 2.72 g of the crystals of the title compound (yield: 69.3%). ¹H-NMR data of these crystals agreed with those obtained in Example 1.

### Example 8

### Production of 1-benzyl-4-[(5,6-dimethoxy-1-indanon)-2-ylidene]methylpiperidine using ethanol and sodium methoxide and adopting method B

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of ethanol. After the air in the system was replaced with nitrogen, 2.4 g (12.4 mmol, 1.2 equivalents) of a 28%-sodium methoxide/methanol solution was poured into the reaction solution with refluxing (79°C). After completion of the pouring, stirring was continued with refluxing for 1 hour and 35 minutes to complete the reaction, whereby crystals were precipitated. The crystals were precipitated during the refluxing. Then, the reaction solution was cooled to 5°C (cooling rate: 32°C/hour). The crystals precipitated at 5°C or higher were collected by filtration, washed with water (30 mL) and 6 mL of methanol and then dried at 50°C (drying time: 2 hours) to obtain 3.67 g of the crystals of the title compound (yield: 93.4%). ¹H-NMR data of these crystals agreed with those obtained in Example 1.

### Example 9

### Production of 1-benzyl-4-[(5,6-dimethoxy-1-indanon)-2-ylidene]methylpiperidine using a mixed solvent of ethanol and toluene and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 mL of a mixed solvent (ethanol: toluene = 16 : 14). After the air in the system was replaced with nitrogen, 2.4 g (12.4 mmol, 1.2 equivalents) of a 28%-sodium methoxide/methanol solution was poured into the reaction solution with refluxing (79°C). After completion of the pouring, stirring was continued with refluxing for 1 hour to complete the reaction. Then, the reaction solution was cooled to 3°C (cooling rate: 25°C/hour). Crystals began to be precipitated at 46°C. The crystals were collected by filtration, washed with water (30 mL) and 6 mL of methanol and then dried at 50°C (drying time: 2 hours) to obtain 3.24 g of the crystals of the title compound (yield: 82.5%). ¹H-NMR data of these crystals agreed with those obtained in Example 1.

### Example 10

### Production of 1-benzyl-4-[(5,6-dimethoxy-1-indanon)-2-ylidene]methylpiperidine using 2-propanol and sodium methoxide and adopting method B

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of 2-propanol. After the air in the system was replaced with nitrogen, 2.4 g (12.4 mmol, 1.2 equivalents) of a 28%-sodium methoxide/methanol solution was poured into the reaction solution at an internal temperature of 60°C. After completion of the pouring, stirring was continued with refluxing (about 80°C) for 1 hour to complete the reaction, whereby crystals were precipitated. The crystals began to be precipitated (precipitation temperature: 70°C) before the refluxing. Then, the reaction solution was cooled (cooling rate: 33°C/hour) and the crystals precipitated at 7°C or higher were collected by filtration. The crystals collected by filtration were washed with water (30 ml) and 6 mL of methanol and dried at 50°C (drying time: 2 hours) to obtain 3.07 g of the crystals of the title compound (yield: 78.20). ¹H-NMR data of these crystals agreed with those obtained in Example 1.

### Example 11

### Production of 1-benzyl-4-[(5,6-dimethoxy-1-indanon)-2-ylidene]methylpiperidine using a mixed solvent of 2-propanol and toluene and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 mL of a mixed solvent (2-propanol : toluene = 16 : 14). After the air in the system was replaced with nitrogen, 2.4 g (12.4 mmol, 1.2 equivalents) of a 28%-sodium methoxide/methanol solution was poured into the reaction solution with refluxing (83°C). After completion of the pouring, stirring was continued with refluxing for 1 hour and 2 minutes to complete the reaction. Then, the reaction solution was cooled to 7°C (cooling rate: 33°C/hour). Crystals began to be precipitated at 42°C. The crystals were collected by filtration, washed with water (30 ml) and 6 mL of methanol and then dried at 50°C (drying time: 1 hour and 20 minutes) to obtain 2.87 g of the crystals of the title compound (yield: 73.1%). ¹H-NMR data of these crystals agreed with those obtained in Example 1.

### Example 12

### Production of 1-benzyl-4-[(5,6-dimethoxy-1-indanon)-2-ylidene]methylpiperidine using 1-propanol and sodium methoxide and adopting method B

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of 1-propanol. After the air in the system was replaced with nitrogen, 2.4 g (12.4 mmol, 1.2 equivalents) of a 28%-sodium methoxide/methanol solution was poured into the reaction solution at an internal temperature of 71°C. After completion of the pouring, stirring was continued with refluxing (about 90°C) for 1 hour to complete the reaction, whereby crystals were precipitated. The crystals began to be precipitated before the refluxing. Then, the reaction solution was cooled (cooling rate: 38°C/hour) and the crystals precipitated at 7°C or higher were collected by filtration. The crystals collected by filtration were washed with water (30 mL) and 6 mL of methanol and dried at 50°C (drying time: 2 hours) to obtain 3.14 g of the crystals of the title compound (yield: 79.90). ¹H-NMR data of these crystals agreed with those obtained in Example 1.

### Example 13

### Production of 1-benzyl-4-[(5,6-dimethoxy-1-indanon)-2-ylidene]methylpiperidine using a mixed solvent of 1-propanol and toluene and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 mL of a mixed solvent (1-propanol : toluene = 16 : 14). After the air in the system was replaced with nitrogen, 2.4 g (12.4 mmol, 1.2 equivalents) of a 28%-sodium methoxide/methanol solution was poured into the reaction solution with refluxing (95°C). After completion of the pouring, stirring was continued with refluxing for 1 hour to complete the reaction. Then, the reaction solution was cooled to 5°C (cooling rate: 38°C/hour). Crystals began to be precipitated at 40°C. The crystals were collected by filtration, washed with water (30 mL) and 6 mL of methanol and then dried at 50°C (drying time: 2 hours and 40 minutes) to obtain 2.51 g of the crystals of the title compound (yield: 63.9%). ¹H-NMR data of these crystals agreed with those obtained in Example 1.

### Example 14

### Production of 1-benzyl-4-[(5,6-dimethoxy-1-indanon)-2-ylidene]methylpiperidine using toluene and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of toluene. After the air in the system was replaced with nitrogen, 2.4 g (12.4 mmol, 1.2 equivalents) of a 28%-sodium methoxide/methanol solution was poured into the reaction solution at 66°C. After completion of the pouring, stirring was continued with refluxing (85°C) for 1 hour to complete the reaction. Then, the reaction solution was cooled (cooling rate: 31°C/hour) and the crystals precipitated at 5°C or higher were collected by filtration. The crystals began to be precipitated at 37°C. The crystals collected by filtration were washed with water (30 mL) and 6 mL of methanol and dried at 50°C (drying time: 2 hours and 25 minutes) to obtain 2.85 g of the crystals of the title compound (yield: 72.60). ¹H-NMR data of these crystals agreed with those obtained in Example 1.

### Example 15

### Production of 1-benzyl-4-[(5,6-dimethoxy-1-indanon)-2-ylidene]methylpiperidine using a mixed solvent of tetrahydrofuran and toluene and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 mL of a mixed solvent (tetrahydrofuran : toluene = 16 : 14). After the air in the system was replaced with nitrogen, 2.4 g (12.4 mmol, 1.2 equivalents) of a 28%-sodium methoxide/methanol solution was poured into the reaction solution with refluxing (79°C). After completion of the pouring, stirring was continued with refluxing for 1 hour to complete the reaction. Then, the reaction solution was cooled (cooling rate: 30°C/hour) and the crystals precipitated at 4°C or higher were collected by filtration. The crystals began to be precipitated at 44°C. The crystals collected by filtration were washed with water (30 mL) and 6 mL of methanol and dried at 50°C (drying time: 2 hours and 25 minutes) to obtain 3.00 g of the crystals of the title compound (yield: 76.4%). ¹H-NMR data of these crystals agreed with those obtained in Example 1.

### Example 16

### Production of 1-benzyl-4-[(5,6-dimethoxy-1-indanon)-2-ylidene]methylpiperidine using a mixed solvent of methanol and toluene and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 mL of a mixed solvent (methanol : toluene = 12 : 18). After the air in the system was replaced with nitrogen, 2.4 g (12.4 mmol, 1.2 equivalents) of a 28%-sodium methoxide/methanol solution was poured into the reaction solution at 60°C. After completion of the pouring, stirring was continued with refluxing (67°C) for 1 hour to complete the reaction. Then, the reaction solution was cooled (cooling rate: 26°C/hour) and the crystals precipitated at 5°C or higher were collected by filtration. The crystals began to be precipitated at 42°C. The crystals collected by filtration were washed with water (30 mL) and 6 mL of methanol and dried at 50°C (1 hour and 20 minutes) and then at 110°C (1 hour and 5 minutes) to obtain 3.49 g of the crystals of the title compound (yield: 88.9%). ¹H-NMR data of these crystals agreed with those obtained in Example 1.

### Example 17

### Production of 1-benzyl-4-[(5,6-dimethoxy-1-indanon)-2-ylidene]methylpiperidine using a mixed solvent of methanol and toluene and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 mL of a mixed solvent (methanol : toluene = 6 : 24). After the air in the system was replaced with nitrogen, 2.4 g (12.4 mmol, 1.2 equivalents) of a 28%-sodium methoxide/methanol solution was poured into the reaction solution at 62°C. After completion of the pouring, stirring was continued with refluxing (about 71°C) for 1 hour to complete the reaction. Then, the reaction solution was cooled (cooling rate: 29°C/hour) and the crystals precipitated at 7°C or higher were collected by filtration. The crystals began to be precipitated at 60°C. The crystals collected by filtration were washed with water (30 mL) and 6 mL of methanol and dried at 50°C (1 hour and 20 minutes) and then at 100°C (1 hour and 5 minutes) to obtain 3.44 g of the crystals of the title compound (yield: 87.6%). ¹H-NMR data of these crystals agreed with those obtained in Example 1.

### Example 18

### Production of 1-benzyl-4-[(5,6-dimethoxy-1-indanon)-2-ylidene]methylpiperidine using ethanol and sodium ethoxide and adopting method B

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of ethanol. After the air in the system was replaced with nitrogen, a solution of 0.85 g (12.5 mmol, 1.2 equivalents) of sodium ethoxide in 4.5 mL of ethanol was poured into the reaction solution with refluxing (75°C). After completion of the pouring, stirring was continued with refluxing (76-79°C) for 1 hour and 12 minutes to complete the reaction, whereby crystals were precipitated. The crystals were precipitated during the refluxing. Then, the reaction solution was cooled (cooling rate: 35°C/hour) and the crystals precipitated at 5°C or higher were collected by filtration. The crystals collected by filtration were washed with water (30 mL) and 6 mL of methanol and dried at 50°C (drying time: 1 hour) to obtain 3.65 g of the crystals of the title compound (yield: 92.9%). ¹H-NMR data of these crystals agreed with those obtained in Example 1.

### Example 19

### Production of 1-benzyl-4-[(5,6-dimethoxy-1-indanon)-2-ylidene]methylpiperidine using ethyl acetate and sodium methoxide and adopting method B

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of ethyl acetate. After the air in the system was replaced with nitrogen, 2.4 g (12.4 mmol, 1.2 equivalents) of a 28%-sodium methoxide/methanol solution was poured into the reaction solution at 65°C. After completion of the pouring, stirring was continued with refluxing (about 71°C) for 1 hour and 5 minutes to complete the reaction. Crystals were precipitated during the refluxing. Then, the reaction solution was cooled (cooling rate: 31°C/hour) and the crystals precipitated at 5°C or higher were collected by filtration. The crystals collected by filtration were washed with water (30 mL) and 6 mL of methanol and dried at 50°C (drying time: 1 hour) to obtain 2.98 g of the crystals of the title compound (yield: 75.9%). ¹H-NMR data of these crystals agreed with those obtained in Example 1.

### Example 20

### Production of 1-benzyl-4-[(5,6-dimethoxy-1-indanon)-2-ylidene]methylpiperidine using a mixed solvent of methanol and toluene and sodium hydroxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 mL of a mixed solvent (methanol : toluene = 16 : 14). After the air in the system was replaced with nitrogen, 0.5 g (12.5 mmol, 1.2 equivalents) of sodium hydroxide was added to the reaction solution with refluxing (65°C). After completion of the addition, stirring was continued with refluxing for 1 hour to complete the reaction. Then, the reaction solution was air-cooled until crystals were precipitated. After the precipitation, the reaction solution was further cooled (cooling rate: 36°C/hour) and the crystals precipitated at 4°C or higher were collected by filtration. The crystals began to be precipitated at 49°C. The crystals collected by filtration were washed with water (30 mL) and 6 mL of methanol and dried at 50°C (drying time: 2 hours and 35 minutes) to obtain 3.31 g of the crystals of the title compound (yield: 84.3%). ¹H-NMR data of these crystals agreed with those obtained in Example 1.

### Example 21

### Production of 1-benzyl-4-[(5,6-dimethoxy-1-indanon)-2-ylidene]methylpiperidine using 1,2-dimethoxyethane and sodium methoxide and adopting method A

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of 1,2-dimethoxyethane. After the air in the system was replaced with nitrogen, 2.4 g (12.4 mmol, 1.2 equivalents) of a 28%-sodium methoxide/methanol solution was poured into the reaction solution at 63°C. After completion of the pouring, stirring was continued with refluxing (80°C) for 1 hour to complete the reaction. Then, the reaction solution was cooled (cooling rate: 52°C/hour) and the crystals precipitated at 5°C or higher were collected by filtration. The crystals began to be precipitated at 48°C. The crystals collected by filtration were washed with water (30 mL) and 6 mL of methanol and dried at 50°C (drying time: 2 hours and 35 minutes) to obtain 2.47 g of the crystals of the title compound (yield: 62.90). ¹H-NMR data of these crystals agreed with those obtained in Example 1.

### Example 22

### Production of 1-benzyl-4-[(5,6-dimethoxy-1-indanon)-2-ylidene]methylpiperidine using a mixed solvent of methanol and 1,2-dimethoxyethane and sodium methoxide and adopting method B

Into a 50-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 24 mL of a mixed solvent (methanol: 1,2-dimethoxyethane = 16 : 14). After the air in the system was replaced with nitrogen, 2.4 g (12.4 mmol, 1.2 equivalents) of a 28%-sodium methoxide/methanol solution was poured into the reaction solution at 60°C. After completion of the pouring, stirring was continued with refluxing (about 68°C) for 1 hour to complete the reaction, whereby crystals were precipitated. The crystals were precipitated during the refluxing. Then, the reaction solution was cooled (cooling rate: 26°C/hour) and the crystals precipitated at 8°C or higher were collected by filtration. The crystals collected by filtration were washed with water (30 mL) and 6 mL of methanol and dried at 50°C (1 hour and 20 minutes) and then at 110°C (1 hour and 30 minutes) to obtain 3.67 g of the crystals of the title compound (yield: 93.40). ¹H-NMR data of these crystals agreed with those obtained in Example 1.

### Comparative Example 1

### Production of 1-benzyl-4-[(5,6-dimethoxy-1-indanon)-2-ylidene]methylpiperidine by the process described in Example 1 in patent document 3

Into a 100-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of tetrahydrofuran. After the air in the system was replaced with nitrogen, 2.4 g (12.4 mmol, 1.2 equivalents) of a 28%-sodium methoxide/methanol solution was added dropwise at 17 to 23°C. After completion of the dropwise addition, the resulting mixture was continuously stirred as it was for 1 hour. After 40 mL of water was added to the reaction solution, the resulting mixture was cooled with ice water, and the crystals precipitated were collected by filtration. The crystals collected by filtration were washed with water (50 mL) and methanol (3 mL x 2) and dried at 24-25°C for 13 hours and 40 minutes to obtain 3.46 g of the crystals of the title compound (yield: 88.10). ¹H-NMR data of these crystals agreed with those obtained in Example 1.

### Comparative Example 2

### Production of 1-benzyl-4-[(5,6-dimethoxy-1-indanon)-2-ylidene]methylpiperidine by the process described in Example 2 in patent document 3

Into a 100-mL four-necked flask were charged 2.0 g (10.4 mmol) of 5,6-dimethoxy-1-indanone, 2.5 g (12.3 mmol, 1.2 equivalents) of 1-benzyl-4-formylpiperidine and 30 mL of tetrahydrofuran, and 2.4 g (12.4 mmol, 1.2 equivalents) of a 28%-sodium methoxide/methanol solution was added dropwise thereto at 37 to 43°C. After completion of the dropwise addition, the resulting mixture was continuously stirred as it was for 1 hour. After 40 mL of water was added to the reaction solution, the resulting mixture was cooled with ice water, and the crystals precipitated were collected by filtration. The crystals collected by filtration were washed with water (50 mL) and methanol (3 mL x 2) and dried at 24-25°C for 13 hours and 40 minutes to obtain 3.44 g of the crystals of the title compound (yield: 87.6%). ¹H-NMR data of these crystals agreed with those obtained in Example 1.

### Evaluation Example 1

### Comparison between the production process of the present invention and the production processes adopted in Comparative Examples with respect to the purity of the desired compound and the content of a by-product

The compound of the structural formula (I) obtained by the production process of the present invention and the compound of the structural formula (I) obtained by carrying out an reproduction test on each of Example 1 and Example 2 in patent document 3 as Comparative Examples 1 and 2, respectively, in the present description were compared with respect to purity and the content of a by-product. High performance liquid chromatography (HPLC) was carried out under the following analysis conditions, and the purity of the compound of the structural formula (I) and the content of the compound of the structural formula (a) were measured on the basis of relative area values. The results are shown in Table 1. In Table 1, N.D. denotes a value lower than the limit of detection. HPLC conditions
Detector: an ultraviolet absorptiometer (measuring
wavelength 290 nm)
Column: YMC-Pack ODS-AM-302, 4.6 mmΦ x 150 mm
Mobile phase: water : acetonitrile :
trifluoroacetic acid = 700 : 300 : 1
Flow rate: 0.7 or 0.8 mL/min
Injecting amount: 10 µl
Column temperature: 35°C
Sample: about 1 mg/mL of the mobile phase

**[Table 1]**

| Test sample | Compound (I) purity (%) | Compound (a) content (%) |
|---|---|---|
| Example 1 | 99.8 | N.D. |
| Example 4 | 99.9 | 0.01 |
| Example 5 | 99.8 | 0.01 |
| Example 7 | 99.9 | 0.01 |
| Example 15 | 99.9 | 0.01 |
| Example 16 | 99.8 | N.D. |
| Example 17 | 99.8 | N.D. |
| Example 20 | 99.9 | 0.02 |
| Example 21 | 99.8 | 0.03 |
| Comparative Example 1 | 99.2 | 0.13 |
| Comparative Example 2 | 99.5 | 0.23 |

As is clear from the above results, the compound of the formula (I) or solvate thereof produced according to the present invention has a reduced content of the compound of.the formula (a) and a good purity.

### Evaluation Example 2

### Comparison between the production process of Example 1 and that of Comparative Example 1 with respect to the yield and purity of the desired compound

The yield and purity of the compound of the formula (I) obtained by the production process of Example 1 were measured and then compared with the yield and purity of the compound of the formula (I) obtained by the production process described in Example 1 in patent document 3, i.e., the production process of Comparative Example 1. The results are shown in Table 2.

**[Table 2]**

| Compound | Yield (%) |
|---|---|
| Example 1 | 89.9% (purity 99.8%) |
| Patent document 3 | 88.1% (purity 99.2%) |

As can be seen from the results shown in Table 2, the yield and purity of the compound of the formula (I) obtained according to the present invention are excellent.

### Advantages of the Invention

As described above in detail, according to the present invention, the compound represented by the formula (I) [1-benzyl-4-[(5,6-dimethoxy-1-indanon)-2-ylidene]methylpiperidine] or a solvate thereof is industrially producible in high yield and high purity. Furthermore, the compound of the formula (I) or solvate thereof produced according to the present invention may be used in a subsequent reaction without a conventional purifying treatment such as column chromatographic purification or recrystallization because the compound or solvate has a reduced content of the compound of the formula (a), a by-product and hence has a good purity.

### INDUSTRIAL APPLICABILITY

According to the present invention, it is possible to produce industrially in high purity the compound of the formula (I) or a solvate thereof, which is usable as a starting material for donepezil hydrochloride, a drug effective for the treatment, prevention and the like of, for example, various senile dementias such as Alzheimer type senile dementia, etc.

## Claims

1. A process for producing 1-benzyl-4-[(5,6-dimethoxy-1-indanon)-2-ylidene]methylpiperidine represented by the formula (I): 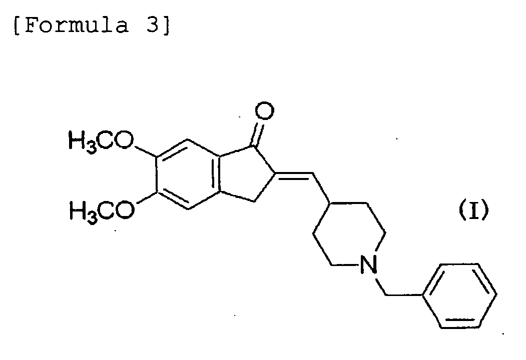 or a solvate thereof, **characterized by** reacting 5,6-dimethoxy-1-indanone represented by the structural formula (III): 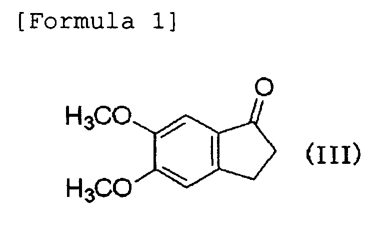 with 1-benzyl-4-formylpiperidine represented by the formula (II): 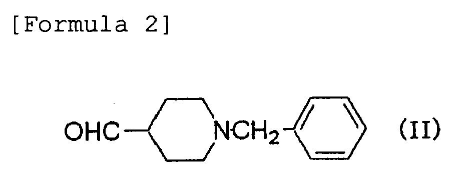 in a solvent in the presence of a base, and then gradually crystallizing the desired compound in the reaction mixture in a high temperature region.

2. A production process according to claim 1, wherein the solvent for reaction is a solvent selected from the group consisting of methanol, ethanol, 1-propanol, 2-propanol, tetrahydrofuran, 1,2-dimethoxyethane, toluene, and mixed solvents thereof.

3. A production process according to claim 1 or 2, wherein the solvent for reaction is methanol, ethanol, tetrahydrofuran, toluene, or a mixed solvent thereof.

4. A production process according to any one of claims 1 to 3, wherein the solvent for reaction is methanol or ethanol.

5. A production process according to any one of claims 1 to 3, wherein the solvent for reaction is tetrahydrofuran. ,

6. A production process according to any one of claims 1 to 3, wherein the solvent for reaction is a mixed solvent of methanol and toluene and the mixing ratio of methanol to toluene is 8 : 2 to 2 : 8.

7. A production process according to any one of claims 1 to 3, wherein the solvent for reaction is a mixed solvent of ethanol and toluene and the mixing ratio of ethanol to toluene is 8 : 2 to 2 : 8.

8. A production process according to any one of claims 1 to 7, wherein the base is an alkali metal hydroxide or an alkali metal alkoxide.

9. A production process according to any one of claims 1 to 8, wherein the base is sodium hydroxide or sodium methoxide.

10. A production process according to any one of claims 1 to 9, wherein the base is sodium methoxide.

11. A production process according to any one of claims 1 to 10, wherein the reaction temperature is 20°C to the reflux temperature of the solvent for reaction.

12. A production process according to any one of claims 1 to 11, wherein the compound of the structural formula (I) or a solvate thereof is obtained while reducing the production of isomers of the desired compound by gradually crystallizing the desired compound in the reaction mixture in a high temperature region.

13. A production process according to claim 12, wherein the isomer of the desired compound is a compound represented by the formula (a) or the formula (b) :

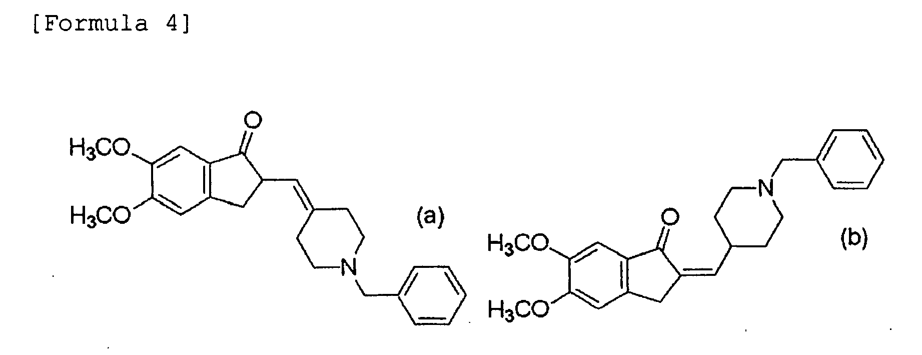

14. A production process according to any one of claims 1 to 13, wherein the high temperature region is a region of 20°C to 95°C.

15. A production process according to any one of claims 1 to 14, wherein the desired compound is crystallized by cooling the reaction mixture slowly from the reaction temperature in the high temperature region to a low temperature region.

16. A production process according to claim 15, wherein the rate of cooling from the reaction temperature in the high temperature region to a low temperature region is 1°C/hour to 30°C/hour.

17. A production process according to any one of claims 1 to 14, wherein the desired compound is crystallized while maintaining the reaction mixture at temperatures in the high temperature region, and then further crystallized by slowly cooling the reaction mixture to a low temperature region.

18. A production process according to claim 17, wherein the rate of cooling from the high temperature region to a low temperature region is 1°C/hour to 60°C/hour.
